(19) Europäisches Patentamt
European Patent Office
Office européen des brevets

(11) **EP 2 831 068 B1**

(12) **EUROPEAN PATENT SPECIFICATION**

(45) Date of publication and mention of the grant of the patent:
**02.03.2016 Bulletin 2016/09**

(21) Application number: **13716179.0**

(22) Date of filing: **28.03.2013**

(51) Int Cl.:
***C07D 451/10*** (2006.01) ***C07D 451/00*** (2006.01)

(86) International application number:
**PCT/CZ2013/000046**

(87) International publication number:
**WO 2013/143510 (03.10.2013 Gazette 2013/40)**

(54) **A METHOD OF PREPARING THE SCOPINE ESTER OF DI-(2-THIENYL)GLYCOLIC ACID, AN INTERMEDIATE IN THE SYNTHESIS OF TIOTROPIUM BROMIDE**

VERFAHREN ZUR HERSTELLUNG VON SCOPINESTER VON DI- (2-THIENYL) GLYKOLSÄURE, EIN ZWISCHENPRODUKT IN DER SYNTHESE VON TIOTROPIUMBROMID

PROCÉDÉ DE PRÉPARATION D'ESTER DE SCOPINE D'ACIDE DI-(2-THIÉNYL)GLYCOLIQUE, UN INTERMÉDIAIRE DANS LA SYNTHÈSE DE BROMURE DE TIOTROPIUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.03.2012 CZ 20120226**

(43) Date of publication of application:
**04.02.2015 Bulletin 2015/06**

(73) Proprietor: **Zentiva, K.S.**
**102 37 Praha 10 (CZ)**

(72) Inventors:
- **CERNA, Igor**
  **067 16 Vyrava (SK)**
- **HAJICEK, Josef**
  **250 81 Nehvizdy (CZ)**

(74) Representative: **Jirotkova, Ivana et al**
**Rott, Ruzicka & Guttmann**
**Patent, Trademark and Law Offices**
**Vinohradskà 37**
**120 00 Praha 2 (CZ)**

(56) References cited:
**EP-A1- 0 418 716** **US-A1- 2007 027 320**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 831 068 B1

## Description

### Technical Field

The invention relates to a new preparation method of the scopine ester of di-(2-thienyl)glycolic acid (hereinafter the scopine ester only) of formula **I.** The scopine ester **I** is an important intermediate in the synthesis of tiotropium bromide, a substance with the chemical name (1R,2R,4S,5S,7S)-7-(2-hydroxy-2,2-di(thiophen-2-yl)acetoxy)-9,9-dimethyl-3-oxa-9-azatricyclo[3.3.1.0$^{2,4}$]nonan-9-ium bromide and the structure **II.**

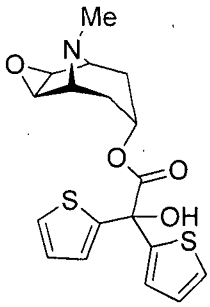

I

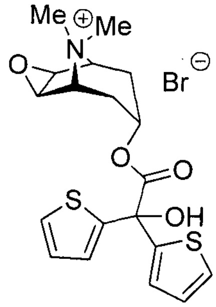

II

### Background Art

Tiotropium bromide (**II**), first described in patent EP 0 418 716, is a selective, competitive, reversible antagonist of cholinergic receptors with a long-term effect. Unlike the structurally similar ipratropium it selectively blocks the muscarinic receptors M1 and M3, while it blocks receptors M2 for a short time. It has significant bronchodilating effects. It is especially used to treat the chronic obstructive pulmonary disease (COPD) and asthma. The therapeutic dosage of the active substance is in micrograms, in the form of powder, which is administered by means of an inhalation device.

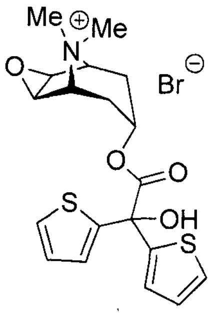

II

With regard to low therapeutic doses of the active substance, the pharmaceutical production of the substance and the process of its preparation is subject to high requirements not only concerning the yield of the process and its economical and environmental aspects, but also requirements for high purity of the resulting active substance.

A preparation process of tiotropium bromide was first published in patent EP 0 418 716 and is illustrated in Scheme 1.

Scheme 1

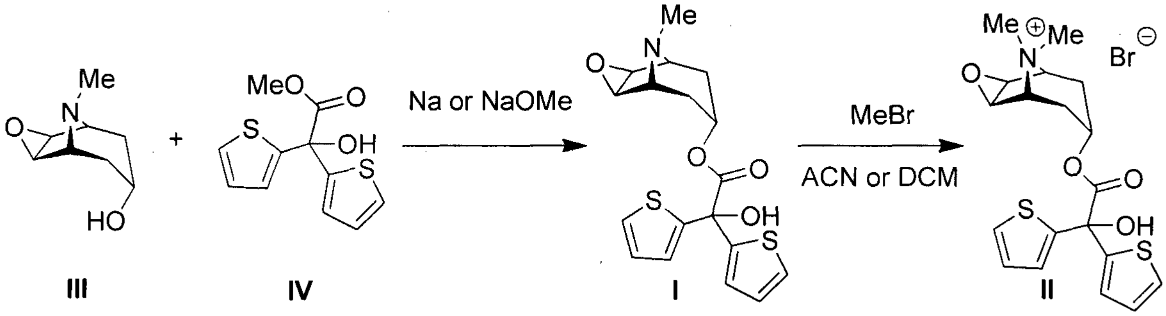


III IV I II

The first step is transesterification of methyl di(2-thienyl)glycolate **(IV)** with scopine **(III)** in a strongly basic environment. The reaction is carried out in an organic solvent (toluene, xylene, or heptane) or preferably in a melt. According to the patent metallic sodium, sodium hydride, sodium methoxide or sodium ethoxide is used as the strong base. The described method uses strongly basic conditions; the amount of the base varies from substoichiometric (0.1 - 0.36 equivalents per mole of scopine) if metallic sodium is used, to stoichiometric amounts (1.0 equivalent per mole of scopine) if sodium methoxide is used as the base. The temperature of the reaction should not exceed 95°C; the reaction is carried out at a reduced pressure (50 kPa to 4 kPa). The reaction mixture is processed by acidification, the acidic phase is subsequently alkalized to prepare a free base, which is extracted with dichloromethane. The product is crystallized from acetonitrile. The yields of the synthesis of the scopine ester **I** vary in the range from 45% to 70%.

As can be seen, the above mentioned examples describing preparation of the scopine ester use harsh conditions that could hardly be used to synthesize the product in the industrial scale.

It is especially the combination of using metallic sodium and carrying out the reaction in a melt at high temperatures and reduced pressure that poses high risks when employed in a larger scale in pharmaceutical production.

The second step is quaternization. The reaction of the scopine ester with methyl bromide is carried out in acetonitrile or in a solvent mixture of dichloromethane and acetonitrile. The subsequent re-crystallization was performed in a not precisely specified mixture of methanol and acetone and a white crystalline product with the melting point of 217 to 218°C was obtained. The patent examples do not mention any yield of quaternization with methyl bromide.

An alternative method of synthesis of tiotropium bromide is described in patent US 6 506 900 B1 and is illustrated in Scheme 2.

Scheme 2

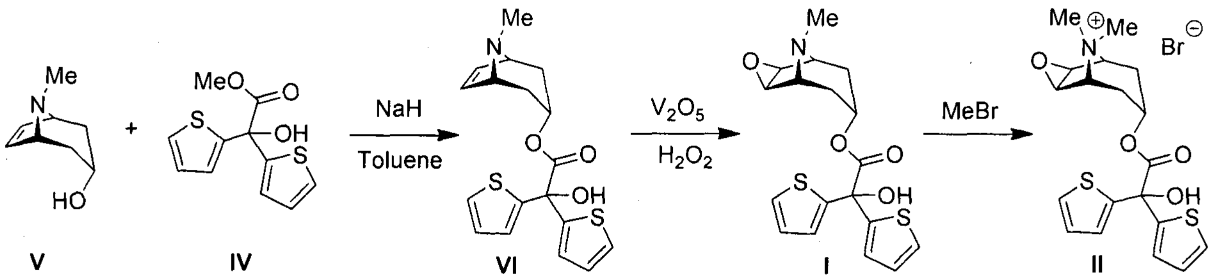


V IV VI I II

It starts with tropenol (**V**), which is used in a reaction with methyl di(2-thienyl)glycolate (**IV**) in the presence of at least the stoichiometric amount of sodium hydride (preferably 1.5-2.0 equivalents per mole of tropenol) at a temperature of 60-75°C and a pressure of 25-30 kPa. The second step is oxidation of the tropenol ester **VI** to the scopine ester **I** with the use of vanadium dioxide and hydrogen peroxide, and the subsequent quaternization provides tiotropium bromide **II** in the total yield of 71%. Even though the selected procedure is referred to as an improvement of the method described in the basic patent, it uses a higher than stoichiometric amount of the strong base for the transesterification and the use of tropenol as the starting compound adds an epoxidation step to the synthesis.

Another patent US 6 747 154 B2 mentions a procedure wherein scopine methobromide **VII** is used instead of scopine in the reaction with di(2-thienyl)glycolic acid **VIII** or its derivatives. The reaction proceeds in a dipolar aprotic solvent (*N*-methylpyrrolidinone, dimethylacetamide) under basic conditions in the presence of the lithium salt of imidazole (Scheme 3).

However, performing the (trans)esterification after the quaternization may not be quite suitable from the point of view of synthetic impurities produced during the (trans)esterification (scopoline, scopoline ester and others), which

can be removed more efficiently if the reverse order is used, namely by purification of the scopine ester after the transesterification and subsequently by crystallization after the quaternization. In addition, no experimental details of using this reaction for synthesis of tiotropium bromide, including yields, are mentioned in the patent.

Scheme 3

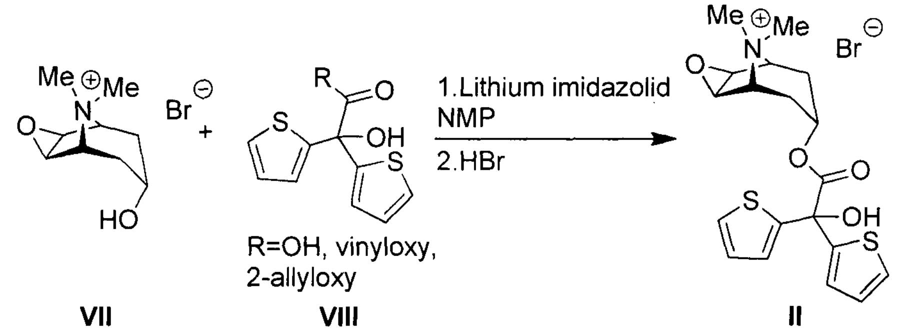


**VII** **VIII** **II**

Application US2006/0047120 presents an improvement of the above mentioned method from the point of view of control of impurities, using protection of the free hydroxyl group of the sodium salt of di(2-thienyl)glycolic acid **IX** with the trimethylsilyl group (Scheme 4). Although this is an *in situ* production of the protected derivative **X,** it increases complexity of the synthesis. The yields of the sequence executed this way vary in the range of 34-85% of the resulting tiotropium bromide **II.**

Scheme 4

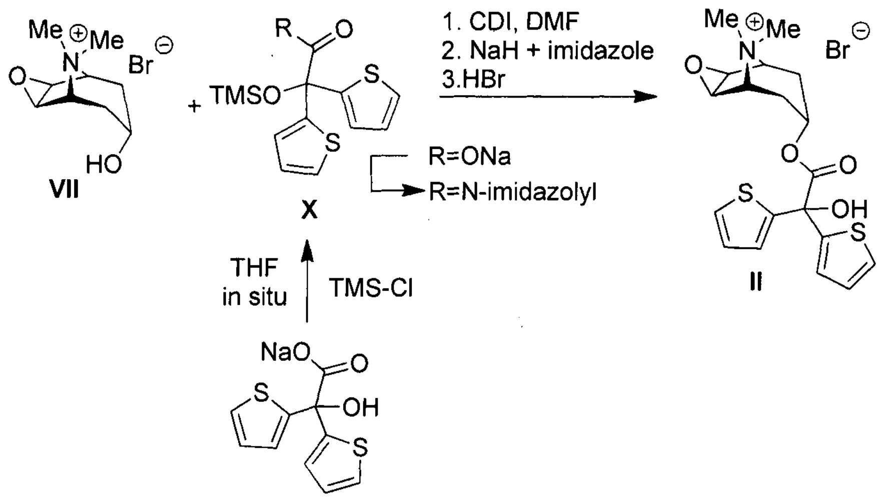


**VII** **X** **II**

**IX**

Patent application WO 2008/008376 describes the transesterification procedure using an excess (2.5 equivalents per mole of scopine hydrobromide, or 1.5 equivalents per mole of scopine) of an anhydrous inorganic base ($K_2CO_3$) in dimethylformamide at the temperature of 65°C and reduced pressure of 7-10 kPa (Scheme 5). The subsequent quaternization with methyl bromide was performed in acetonitrile at the room temperature. The total yield of tiotropium bromide was 42-61 %.

Scheme 5

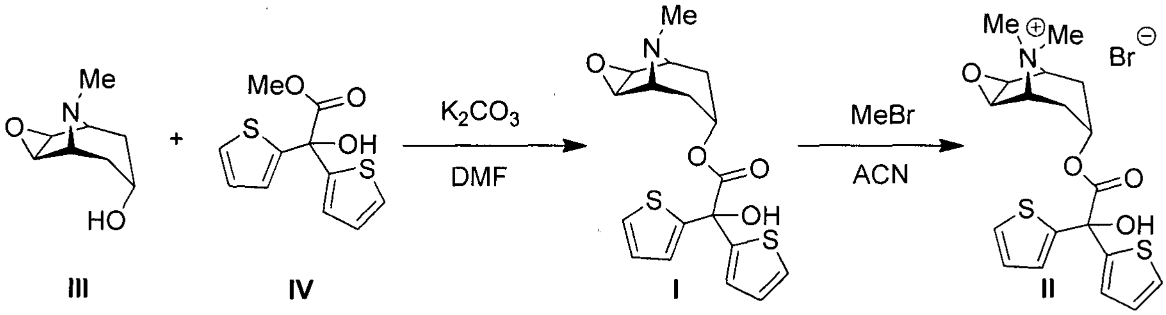


III IV I II

In patent application WO 2009/087419 scopine **III** is used for the transesterification of methyl di(2-thienyl)glycolate **IV** in the hydrochloride salt form (Scheme 6). The reaction is performed in DMF at 60°C in basic conditions in the presence of an organic amine (1 to 3 equivalents of 1,8-diazabicyclo[5.4.0]undec-7-ene; DBU), preferably in combination with another base (1 to 2 equivalents of NaH per mole of scopine hydrochloride). The subsequent quaternization with methyl bromide can also be carried out without isolation of the scopine ester **II**, but such a procedure provides the resulting product in an unsuitable purity and low yield. The total yield is 41 to 50%.

Scheme 6

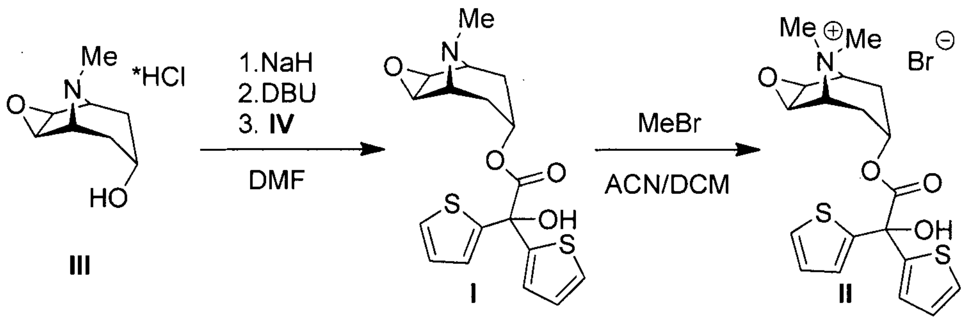


III I II

This summary of patent literature clearly documents the need of a simple, economically and environmentally acceptable method of synthesis of tiotropium bromide, applicable also in a larger scale with sufficient control over possibly produced impurities.

**Disclosure of Invention**

The invention provides a new efficient method of preparing tiotropium bromide **II**

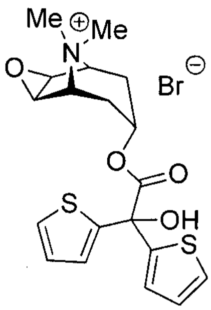

**II**

which comprises the following steps:

a) preparation of the scopine ester of formula **I**

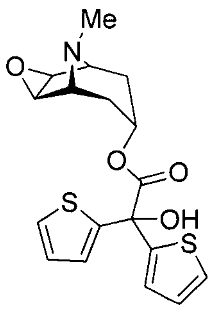

I

by transesterification of methyl di(2thienyl)glycolate of formula **IV** with scopine of formula **III**

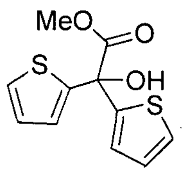

IV

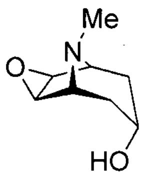

III

in the presence of a sterically hindered base selected from the group of alkali salts of branched $C_3$ to $C_5$ alkoxides in an inert solvent,

b) isolation of the scopine ester of formula **I**, and

c) quaternization of the scopine ester of formula **I** with methyl bromide.

The new method of synthesis of tiotropium bromide is shown in Scheme 7 below.

Scheme 7

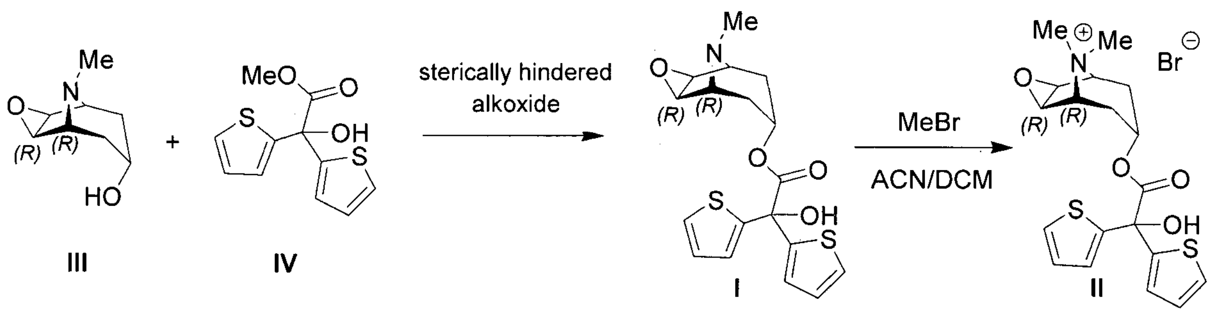


III IV I II

For the transesterification, a sterically hindered base has been selected that in substoichiometric amounts (0.3-0.7 equivalents per mole of scopine) provably reduces the risk of formation of the undesired scopoline **XI** (observed when scopine was subjected to the effect of a strong base, Scheme 8) and its derivative scopoline ester **XII,** a side product of the transesterification.

Scheme 8

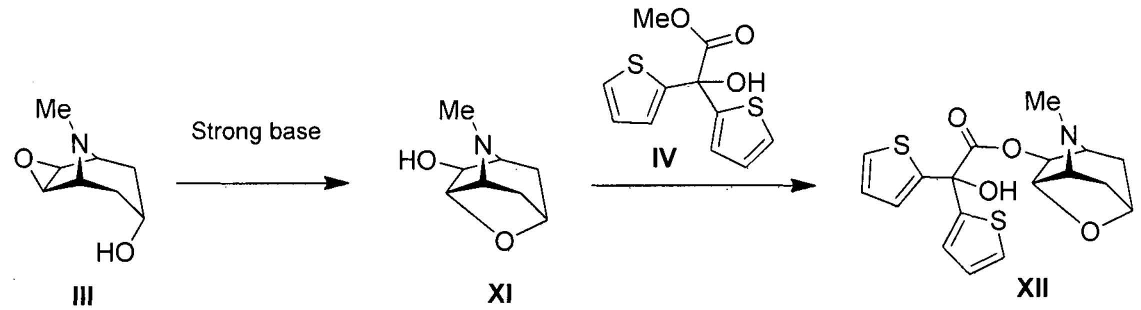


III XI XII

Table 1 presents examples of carrying out the transesterification under different conditions. The first three examples of Table 1 are reproductions of patent EP0418716. Example 1 reproduces an example of the patent EP041876 with the use of metallic sodium, which was added in parts into a stirred solution of scopine **III** and the reagent **IV** in toluene at 90°C. After acidic-basic processing a crude mixture was obtained that contained 81% of the scopine ester and 18% of impurities derived from scopoline **(XI** and **XII).** Crystallization from acetonitrile yielded 50% of product **I** with the purity of 98.75% (analyzed by UPLC). Reproduction of the experiment of the patent EP0418716 using other bases such as sodium hydride, and especially sodium methoxide, which would be much more acceptable than metallic sodium for the synthesis from the point of view of carrying out the reaction in a larger scale, showed that the course of the reaction was very unselective and unpredictable with a high proportion of scopoline impurities **XI** and **XII** in the crude reaction mixture (66.1% of **XI** and **XII** when using NaH; and 58.7% of **XI** and **XII** in the case of using sodium methoxide, respectively, Examples 2, 3).

It would be a great advantage to use a base that would be available, preferably easy to handle and safe from the point of view of using in a larger scale and, last but not least, sufficiently selective. All these conditions are fulfilled by the use of a non-nucleophilic sterically hindered alkoxide.

In the Examples the use of sodium *tert*-butoxide or *tert*-pentoxide in the stoichiometric amounts (Examples 4, 5) resulted in a significant increase of selectivity (~51% of the product **I** and ~42% of the impurities **XI** and **XII** in the crude reaction mixture) as compared to the use of the related sodium methoxide (11.6% of the product **I** and 58.7% of the impurities **XI** and **XII** in the crude reaction mixture, Example 3).

In further experiments we focused on the amount of the sterically hindered alkoxide used. It has been found out that by using a substoichiometric amount of the sterically hindered alkoxide (0.3-0.5 equivalents per mole of scopine **III,** sodium or potassium *tert*-butoxide, Examples 6 and 7) acceptable values of scopoline impurities can be achieved (~25%) while maintaining nearly 100% conversion of the reaction. In addition, in a preferable embodiment of the reaction with the use of 0.5 equivalents of sodium *tert*-butoxide (Example 8) the crude mixture contained 84.7% of the scopine ester **I** and only 11.4% of the impurities **XI** and **XII.** Crystallization of the crude product from acetonitrile provided 58% of the product **I** with the purity of 99.2% (analyzed by UPLC).

Thus, the use of a substoichiometric amount of a sterically hindered base represents a very convenient alternative to the method using metallic sodium (or other bases mentioned in the patent literature), from the point of view of both the process safety and a higher yield, process selectivity and purity of the final product **I.**

Table 1

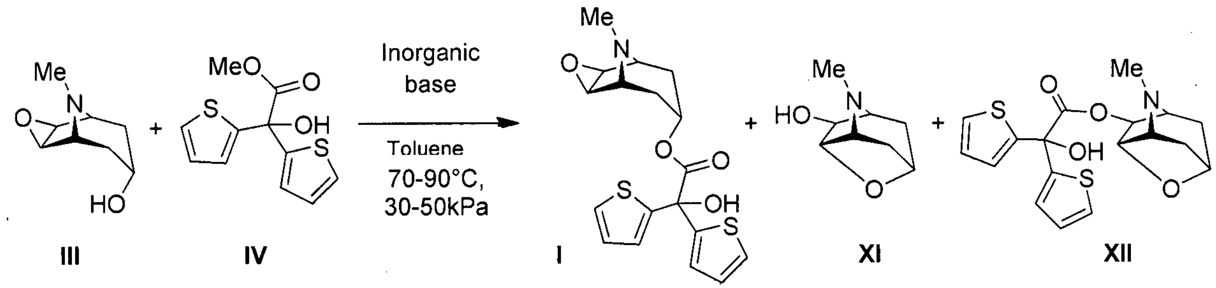


III + IV → I + XI + XII

| Example | Base (mole equivalent) | Analysis of the crude reaction mixture (after processing) with gas chromatography | | | | |
|---|---|---|---|---|---|---|
| | | III | IV | I | XI | XII |
| 1 | Na(0,36) | <0.5 % | 0.85 % | 81.0 % | 4.1 % | 14.0 % |
| 2 | NaH(1) | <0.5 % | 0.5 % | 24.2 % | 35.7 % | 30.4 % |
| 3 | NaOMe(1) | 17.8 % | 5.3 % | 11.6 % | 57.3 % | 1.4 % |
| 4 | t-pentONa(1) | <0.5 % | 1.0 % | 49.6 % | 32.5 % | 12.1 % |
| 5 | t-BuONa(1) | <0.5 % | 0.6 % | 53.0 % | 41.7 % | 1.0 % |
| 6 | t-BuOK(0.5) | <0.5 % | 0.5 % | 72.0 % | 18.4 % | 5.6 % |
| 7 | t-BuONa(0.3) | 0.5 % | 0.8 % | 67.5 % | 26.3 % | 1.5 % |
| 8 | t-BuONa(0.5) | <0.05 % | <0.05 % | 84.7 % | 5.3 % | 6.1 % |

The term sterically hindered base means a sterically hindered alkoxide such as *tert*-butoxide, *tert*-pentoxide, isopropoxide, or benzoxide, in the form of a salt with an alkali metal. In a preferable embodiment the sodium or potassium salt of *tert*-butoxide or *tert*-pentoxide, especially *tert*-butoxide, is used.

The transesterification is carried out in an inert organic solvent such as aromatic hydrocarbons or cyclic ethers, especially toluene or tetrahydrofuran, or their mixtures.

1-1.05 equivalents of methyl di(2-thienyl)glycolate **IV** per mole of scopine **III** are used for the reaction, in a preferable embodiment 1 equivalent of **IV** is used.

The reaction is carried out by slow addition of the base to a stirred solution of the starting compounds **III** and **IV** in a suitable solvent. The base is added by adding a solution of the base in a suitable solvent dropwise; preferably a 2M solution of sodium *tert*-butoxide in tetrahydrofuran or in toluene is used. The addition of the base is carried out at a temperature of 50 to 90°C, preferably at the temperature of 70°C and a rate of 7-8 g/30 min.

The reaction itself is carried out in the temperature range of 80 to 130°C, preferably at 90 °C and a pressure in the range of 50 kPa to 30 kPa, under occasional removal of methanol from the reaction mixture by distillation, for 3-6 hours, preferably for 4 hours. The term isolation in step b refers to admixing of the reaction mixture, after possible dilution with an extraction solvent, to a cooled aqueous solution of an inorganic acid; in a preferable embodiment hydrochloric acid is used. By shaking the acidic aqueous phase with the extraction solvent the salt of the scopine ester **I** is freed from most organic impurities (unreacted residues of the starting compounds, scopoline). Dichloromethane is a suitable extraction solvent.

The aqueous layer containing the salt of the scopine ester **I** is alkalized with a suitable base to pH in the range of 7-9, preferably 8-9; preferably, an aqueous solution of sodium carbonate is used as the base. The product is subsequently extracted with an extraction solvent, dichloromethane being preferably used as the solvent.

This way the product scopine ester **I** is obtained in the purity of 80.0-85.0% (determined by gas chromatography), preferably in a higher purity than 80% (determined by gas chromatography).

Crystallization of thus obtained crude product is carried out in a suitable solvent, acetonitrile or toluene and their mixtures, preferably acetonitrile, being used. The crystallization is carried out from a solution of the scopine ester **I** in acetonitrile at -5°C to -50°C, preferably at -10 to -40°C. The crystalline product is isolated and dried at the normal pressure or *in vacuo* at temperatures in the range from the room temperature to 50°C.

The product **I** is isolated in the purity of 98.5% to 99.5%, preferably in purity higher than 99.0%.

The second step of synthesis of tiotropium bromide **II** is quaternization of the scopine ester **I** with methyl bromide, which is carried out analogously to the procedure described in the basic patent EP0418714.

## Examples

The melting temperatures were measured using a Kofler block.

Method of analyzing the crude reaction mixture by gas chromatography

Gas chromatography (2.2.28) with direct injection and FI detection is applied:

*Chromatographic conditions:*

*Capillary column:* Rtx-1 (30 m, 0.25 mm ID, 0.25 μm df) or equivalent

*Temperature program:* 60°C (0 min), gradient 15°C/min to 105°C (0 min), gradient 5°C/min to 135°C (0 min), gradient 20°C/min to 245°C (0 min), gradient 5°C/min to 280°C (0 min), gradient 20°C/min to 320°C (8 min)

| | |
|---|---|
| *Carrier gas:* | *helium for chromatography* R; 40 cm/s, constant flow |
| *Injection:* | 1 μl |
| *Injector:* | 250°C, splitting ratio 10:1 |
| *Detector:* | FID, 330°C |

*Preparation of solutions:*

*Blank sample:* 1 ml of *dichloromethane* is transferred into a 2ml GC vial and the vial is closed.

*Tested sample:* The amount of 10 mg of the tested substance is dissolved in 1 ml of dichloromethane in a 2ml GC vial and the vial is closed.

Approximate retention times:

| | |
|---|---|
| Scopoline | 6.6 min |
| Scopine | 7.2 min |
| Methyl (di-2-thienyl)glycolate | 12.9 min |
| Scopoline ester | 18.5 min |
| Scopine ester | 18.8 min |

*Evaluation:*

The contents of individual impurities expressed in % are evaluated using the method of internal normalization of peak areas in accordance with the formula:

$$x = \frac{A_x}{\sum_{i=1}^{n} A_i} \times 100 ,$$

wherein
$A_x$ area of the impurity peak on the chromatogram of the sample of the tested substance
$\sum_{i=1}^{n} A$ sum of the areas of all the peaks on the chromatogram of the sample of the tested substance, except the solvent peak

UPLC analysis method of the scopine ester I

Mobile phase:

A: Potassium hydrogen phosphate 0.01 M, pH 7.8 ± 0.05
B: acetonitrile R
Column: UPLC BEH Shield RP 18 or equivalent
Elution: gradient

| Time (min) | % A | % B |
|---|---|---|
| 0.0 | 80 | 20 |
| 5.0 | 30 | 70 |
| 7.0 | 30 | 70 |
| 7.5 | 80 | 20 |

Flow rate: 0.4 ml/min.
Column temperature 15°C
Autosampler temperature: 20°C
Injection: 1 μl
Analysis time: 7.5 min
Equilibration time: 2.5 min
Detection: 238 nm

*Sample preparation:*

25.0 mg of the tested substance is dissolved in an acetonitrile-water (1+1) solvent. It is put in an ultrasonic bath

for 1 min and, after cooling to the laboratory temperature, diluted to 25.0 ml with the same solvent.

Preparation of the scopine ester **I**

**Example 1** (Reference example reproducing the method of patent EP0418716)

1.0 g (6.44 mmol) of scopine and 1.64 g of methyl di(2-thienyl)glycolate (1 equivalent, 6.44 mmol) are weighed into a flask and the mixture is dissolved in 5 ml of toluene at 90°C. 53 mg of sodium (0.36 equivalents, 2.32 mmol) are added in parts to the solution of the reaction mixture stirred at 90°C in the course of 15 min. After the addition the reaction mixture was further stirred at 90°C and at a pressure of 30-10 kPa for 5 h, methanol being occasionally removed by distillation. The reaction mixture is subsequently admixed to a mixture of ice and 2M HCl. The acidic phase is separated, alkalized with 2M $Na_2CO_3$ and the free base of the scopine ester **I** is extracted with dichloromethane. After drying with $Na_2SO_4$ dichloromethane is evaporated at reduced pressure. The crude mixture (1.84 g) contained 81.0% of the scopine ester **I,** 4.1% of scopoline **XI** and 14.0% of scopoline ester **XII** (analyzed by gas chromatography). The crude reaction mixture was dissolved in hot condition in acetonitrile (20 ml), filtered, concentrated to the volume of 10 ml, cooled to -32°C and left at this temperature without being stirred for 20 h. The crystallized product was filtered, washed with a minimum amount of cooled acetonitrile and dried freely at the room temperature for 22 hours and then in a vacuum drier at the room temperature for 3 h. 1.23 g of beige crystals of the scopine ester **I** were obtained in the purity of 98.75% (analyzed by UPLC) and the yield of 50%.

**Example 2** (Reference example reproducing the method of patent EP0418716)

0.50 g (3.22 mmol) of scopine and 0.82 g of methyl di(2-thienyl)glycolate (1 equivalent, 3.22 mmol) are weighed into a flask and the mixture is dissolved in 2.5 ml of toluene at 70°C. A suspension of sodium hydride (0.13 g, 60% dispersion of NaH in mineral oil, 1 equivalent, 3.22 mmol) in toluene (2.5 ml) is added dropwise to the solution of the reaction mixture stirred at 70°C and under inert argon atmosphere during 15 min. After the addition the reaction mixture was further stirred at 90°C and at a pressure of 40-30 kPa for 4 h, methanol being occasionally removed by distillation. The reaction mixture is subsequently admixed to a mixture of ice and 2M HCl. The acidic phase is separated, alkalized with 2M $Na_2CO_3$ and the free base of the scopine ester **I** is extracted with dichloromethane. After drying with $Na_2SO_4$ dichloromethane is evaporated at reduced pressure. The crude mixture (1.01 g) contained 24.2% of the scopine ester **I,** 35.7% of scopoline **XI** and 30.4% of scopoline ester **XII** (analyzed by gas chromatography). The crude reaction mixture was dissolved in hot condition in acetonitrile, filtered, cooled to -32°C and left without being stirred at this temperature for 24 h. The crystallized product was filtered, washed with a minimum amount of cooled acetonitrile and dried in a vacuum drier at the room temperature for 20 hours. 0.26 g of the scopine ester **I** was obtained in the purity of 98.71 % (analyzed with UPLC) and the yield of 21 %.

**Example 3** (Reference example reproducing the method of patent EP0418716)

0.50 g (3.22 mmol) of scopine and 0.82 g of methyl di(2-thienyl)glycolate (1 equivalent, 3.22 mmol) are weighed into a flask and the mixture is dissolved in 4 ml of toluene at 70°C. 174 g of sodium methoxide (1 equivalent, 3.22 mmol) are gradually added in parts to the solution of the reaction mixture stirred at 90°C in the course of 15 min. After the addition the reaction mixture was further stirred at 90°C and a pressure of 30-10 kPa for 5 h, methanol being occasionally removed by distillation. The reaction mixture is subsequently admixed to a mixture of ice and 2M HCl. The acidic phase is separated, alkalized with 2M $Na_2CO_3$ and the free base of the scopine ester **I** is extracted with dichloromethane. After drying with $Na_2SO_4$ dichloromethane is evaporated at reduced pressure. The crude mixture (0.18 g) contained 11.6% of the scopine ester **I,** 57.3% of scopoline **XI** and 1.4% of scopoline ester **XII** (analyzed by gas chromatography).

**Example 4**

0.50 g (3.22 mmol) of scopine and 0.82 g of methyl di(2-thienyl)glycolate (1 equivalent, 3.22 mmol) are weighed into a flask and the mixture is dissolved in 3 ml of toluene at 70°C. A solution of sodium *tert*-pentoxide (0.37 g, 1 equivalent, 3.22 mmol) in toluene (6 ml) is added dropwise to the solution of the reaction mixture stirred at 70°C under inert argon atmosphere during 15 min. After the addition the reaction mixture was further stirred at 70°C and a pressure of 40-30 kPa for 4.5 h, methanol being occasionally removed by distillation. The reaction mixture is subsequently admixed to a mixture of ice and 2M HCl. The acidic phase is separated, alkalized with 2M $Na_2CO_3$ and the free base of the scopine ester **I** is extracted with dichloromethane. After drying with $Na_2SO_4$ dichloromethane is evaporated at reduced pressure. The crude mixture (0.80 g) contained 49.6% of the scopine ester **I,** 32.5% of scopoline **XI** and 12.1% of scopoline ester **XII** (analyzed by gas chromatography). The crude reaction mixture was dissolved in hot condition in acetonitrile, filtered,

cooled to -32°C and left without being stirred at this temperature for 48 h. The crystallized product was filtered, washed with a minimum amount of cooled acetonitrile and dried in a vacuum drier at the room temperature for 20 hours. 0.30 g of the scopine ester **I** was obtained in the purity of 98.75% (analyzed by UPLC), melting point 149.2-150.3°C, with the yield of 25%.

**Example 5**

0.50 g (3.22 mmol) of scopine and 0.82 g of methyl di(2-thienyl)glycolate (1 equivalent, 3.22 mmol) are weighed into a flask and the mixture is dissolved in 3 ml of toluene at 70°C. A solution of sodium *tert*-butoxide (0.32 g, 1 equivalent, 3.22 mmol) in toluene (6 ml) is added dropwise to the solution of the reaction mixture stirred at 70°C under inert argon atmosphere during 15 min. After the addition the reaction mixture was further stirred at 90°C and a pressure of 40-30 kPa for 4.5 h, methanol being occasionally removed by distillation. The reaction mixture is subsequently admixed to a mixture of ice and 2M HCl. The acidic phase is separated, alkalized with 2M $Na_2CO_3$ and the free base of the scopine ester **I** is extracted with dichloromethane. After drying with $Na_2SO_4$ dichloromethane is evaporated at reduced pressure. The crude mixture (0.59 g) contained 53.0% of the scopine ester **I**, 41.7% of scopoline **XI** and 1.0% of scopoline ester **XII** (analyzed by gas chromatography). The crude reaction mixture was dissolved in acetonitrile, and cooled to -10°C. The crystallized product was filtered, washed with a minimum amount of cooled acetonitrile and dried in a vacuum drier at the room temperature for 20 hours. 0.23 g of the scopine ester **I** was obtained in the purity of 99.08% (analyzed by UPLC), melting point 150.2-150.4°C, with the yield of 19%.

**Example 6**

0.50 g (3.22 mmol) of scopine and 0.82 g of methyl di(2-thienyl)glycolate (1 equivalent, 3.22 mmol) are weighed into a flask and the mixture is dissolved in 3 ml of toluene at 70°C. A solution of potassium *tert*-butoxide (0.18 g, 0.5 equivalents, 1.61 mmol) in toluene (3 ml) is added dropwise to the solution of the reaction mixture stirred at 70°C under inert argon atmosphere during 15 min. After the addition the reaction mixture was further stirred at 90°C and a pressure of 40-30 kPa for 4 h, methanol being occasionally removed by distillation, and in the end the solution was concentrated by distilling toluene off, and diluted with dichloromethane (10 ml). The solution is subsequently admixed to a mixture of ice and 2M HCl. The acidic phase is separated, alkalized with 2M $Na_2CO_3$ and the free base of the scopine ester **I** is extracted with dichloromethane. After drying with $Na_2SO_4$ dichloromethane is evaporated at reduced pressure. The crude mixture (0.82 g) contained 72.0% of the scopine ester **I**, 18.4% of scopoline **XI** and 5.6% of scopoline ester **XII** (analyzed by gas chromatography). The crude reaction mixture was dissolved in acetonitrile, and cooled to -10°C. The crystallized product was filtered, washed with a minimum amount of cooled acetonitrile and dried in a vacuum drier at 45°C for five hours. 0.50 g of the scopine ester **I** was obtained, melting point 149.5-150.9°C, with the yield of 41%.

**Example 7**

0.50 g (3.22 mmol) of scopine and 0.82 g of methyl di(2-thienyl)glycolate (1 equivalent, 3.22 mmol) are weighed into a flask and the mixture is dissolved in 3 ml of toluene at 70°C. A solution of sodium *tert*-butoxide (0.09 g, 0.3 equivalents, 0.97 mmol) in toluene (3 ml) is added dropwise to the solution of the reaction mixture stirred at 70°C under inert argon atmosphere during 15 min. After the addition the reaction mixture was further stirred at 90°C and a pressure of 40-30 kPa for 4 h, methanol being occasionally removed by distillation, and in the end the solution was concentrated by distilling toluene off, and diluted with dichloromethane (10 ml). The solution is subsequently admixed to a mixture of ice and 2M HCl. The acidic phase is separated, alkalized with 2M $Na_2CO_3$ and the free base of the scopine ester **I** is extracted with dichloromethane. After drying with $Na_2SO_4$ dichloromethane is evaporated at reduced pressure. The crude mixture (0.84 g) contained 67.5% of the scopine ester **I**, 26.3% of scopoline **XI** and 1.5% of scopoline ester **XII** (analyzed by gas chromatography). The crude reaction mixture was dissolved in acetonitrile, and cooled to -10°C. The crystallized product was filtered, washed with a minimum amount of cooled acetonitrile and dried in a vacuum drier at 45°C for five hours. 0.41 g of the scopine ester **I** was obtained, melting point 150.3-151.0 °C, with the yield of 33%.

**Example 8**

19.9 g (128.4 mmol) of scopine and 32.7 g methyl di(2-thienyl)glycolate (1 equivalent, 128.4 mmol) are weighed into a flask and the mixture is dissolved at 70°C in 120 ml of toluene. A 2M solution of sodium *tert*-butoxide in tetrahydrofuran (32.1 ml, 0.5 equivalents, 64.2 mmol) is added dropwise to the solution of the reaction mixture stirred at 70 °C under inert argon atmosphere during 25 min. After the addition the reaction mixture was further stirred at 90°C and a pressure of 50 kPa for 4 hours, methanol being occasionally removed by distillation, in the end, the reaction mixture was concentrated to 55 ml by distilling toluene off at the pressure of 30 kPa, and diluted with dichloromethane (400 ml).

Then, the solution is admixed to a mixture of 200 g of ice and 300 ml of 1M HCl. The acidic phase is separated, alkalized with 2M $Na_2CO_3$ and the free base of the scopine ester **I** is extracted with dichloromethane (3x 500 ml each). After drying with $Na_2SO_4$ dichloromethane is evaporated at reduced pressure. The crude mixture (37.2 g) contained 84.7% of the scopine ester **I**, 5.3% of scopoline **XI** and 6.1% of scopoline ester **XII** (analyzed by gas chromatography). The crude reaction mixture was dissolved in acetonitrile (120 ml) and cooled to -10°C. The crystallized product was filtered, washed with a minimum amount of cooled acetonitrile (30 ml) and dried in a vacuum drier at the temperature of 45°C for five hours. 28.1 g of the scopine ester I was obtained in the purity of 99.2% (analyzed by gas chromatography), melting point 150.2-151.3 °C, and in the yield of 58%.

Quaternization of the scopine ester **I** with methyl bromide

**Example 9** (Reference example reproducing the method of patent EP0418716)

23.6 g of the scopine ester **I** (39.7 mmol) was dissolved in 230 ml of a mixture of dry dichloromethane (90 ml) and dry acetonitrile (140 ml) at 55°C, then the solution was cooled to 33°C and 54.0 g of 55% MeBr in dry acetonitrile (5.0 equivalents) were added. The solution was left to cool down freely, and subsequently stirred at the room temperature for 19 hours. The resulting crystalline product was filtered, washed with 900 ml of dichloromethane and dried in a vacuum drier (5 kPa) at 35 °C for 17 hours. 30.8 g of white crystals were obtained, UPLC purity 99.74%. Solvent content (determined by gas chromatography): dichloromethane 67000 ppm, acetonitrile 4300 ppm.

## Claims

**1.** A method of preparing tiotropium bromide of formula **II**

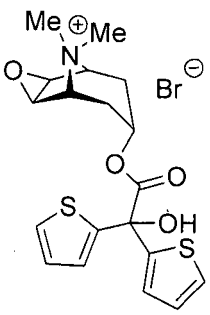

**II**

**characterized in that** it comprises the following steps:

a) preparation of the scopine ester of formula **I**

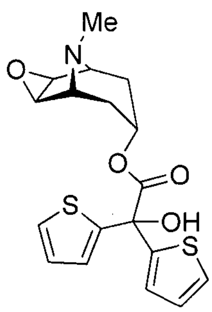

**I**

by transesterification of methyl di(2thienyl)glycolate of formula **IV** with scopine of formula **III**

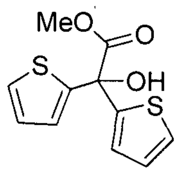

IV

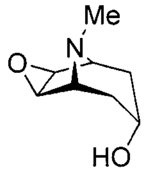

III

in the presence of a substoichiometric amount of a sterically hindered base selected from the group of alkali salts of branched $C_3$ to $C_5$ alkoxides in an inert solvent,
b) isolation of the scopine ester of formula **I**, and
c) quaternization of the scopine ester of formula **I** with methyl bromide.

2. The method according to claim 1, **characterized in that** the sterically hindered base is selected from the group of the sodium or potassium salts of *tert*-butoxide, *tert*-pentoxide, and isopropoxide.

3. The method according to claim 2, **characterized in that** the sterically hindered base is the sodium or potassium salt of *tert*-butoxide or *tert*-pentoxide.

4. The method according to claim 1, **characterized in that** the sterically hindered base is used in the range of 0.3 to 0.7 equivalents related to scopine.

5. The method according to claim 1, **characterized in that** toluene, tetrahydrofuran or their mixtures are used as the inert solvent.

6. The method according to claims 1-5, **characterized in that** the transesterification is carried out at a temperature in the range of 80 to 130°C.

7. The method according to claim 6, **characterized in that** the transesterification is carried out at a pressure in the range of 50 kPa to 30 kPa, the formed methanol being simultaneously removed by distillation.

8. The method according to claim 1, **characterized in that** the isolation in step (b) comprises mixing of the reaction mixture from step (a), optionally after dilution with an extraction solvent, into an aqueous solution of an inorganic acid, extraction of the aqueous phase with an extraction solvent, alkalization of the aqueous phase with an inorganic base to a pH in the range of 7-9, extraction of the product with an extraction solvent and, after concentration, crystallization of the crude scopine ester of formula **I** from the crystallizing solvent.

9. The method according to claim 8, **characterized in that** dichloromethane is used as the extraction agent.

10. The method according to claim 8, **characterized in that** the crystallizing solvent is acetonitrile or toluene or their mixtures.

**Patentansprüche**

1. Verfahren zur Herstellung von Tiotropiumbromid der Formel **II**

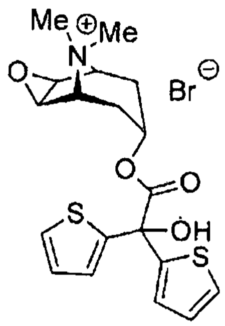

II

**dadurch gekennzeichnet, dass** es die folgenden Schritte umfasst:

a) Herstellung des Scopinesters der Formel **I**

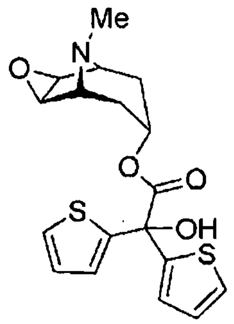

I

durch Umesterung von Methyldi(2thienyl)glycolat der Formel **IV** mit Scopin der Formel **III**

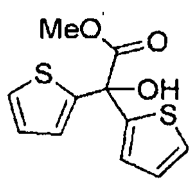

IV

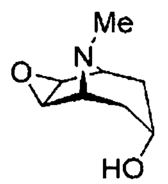

III

in Gegenwart einer unterstöchiometrischen Menge einer sterisch gehinderten Base, ausgewählt aus der Gruppe der Alkalisalze von verzweigten $C_3$- bis $C_5$-Alkoxiden, in einem inerten Lösungsmittel,
b) Isolation des Scopinesters der Formel **I** und
c) Quaternisierung des Scopinesters der Formel **I** mit Methylbromid.

**2.** Verfahren nach Anspruch 1, **dadurch gekennzeichnet, dass** die sterisch gehinderte Base ausgewählt ist aus der Gruppe der Natrium- oder Kaliumsalze von *tert*-Butoxid, *tert*-Pentoxid und Isopropoxid.

**3.** Verfahren nach Anspruch 2, **dadurch gekennzeichnet, dass** die sterisch gehinderte Base das Natrium- oder Kaliumsalz von *tert*-Butoxid oder *tert*-Pentoxid ist.

**4.** Verfahren nach Anspruch 1, **dadurch gekennzeichnet, dass** die sterisch gehinderte Base im Bereich von 0,3 bis 0,7 Äquivalenten, bezogen auf Scopin, verwendet wird.

**5.** Verfahren nach Anspruch 1, **dadurch gekennzeichnet, dass** Toluol, Tetrahydrofuran oder deren Mischungen als

inertes Lösungsmittel verwendet werden.

6. Verfahren nach den Ansprüchen 1-5, **dadurch gekennzeichnet, dass** die Umesterung bei einer Temperatur im Bereich von 80 bis 130 °C durchgeführt wird.

7. Verfahren nach Anspruch 6, **dadurch gekennzeichnet, dass** die Umesterung bei einem Druck im Bereich von 50 kPa bis 30 kPa durchgeführt wird, wobei das gebildete Methanol gleichzeitig abdestilliert wird.

8. Verfahren nach Anspruch 1, **dadurch gekennzeichnet, dass** die Isolation in Schritt (b) das Zumischen des Reaktionsgemischs aus Schritt (a), gegebenenfalls nach Verdünnung mit einem Extraktionslösungsmittel, zu einer wässrigen Lösung einer anorganischen Säure, die Extraktion der wässrigen Phase mit einem Extraktionslösungsmittel, das Alkalisieren der wässrigen Phase mit einer anorganischen Base auf einen pH-Wert im Bereich von 7-9, die Extraktion des Produktes mit einem Extraktionslösungsmittel und, nach Aufkonzentrieren, die Kristallisation des rohen Scopinesters der Formel **I** aus dem Kristallisationslösungsmittel umfasst.

9. Verfahren nach Anspruch 8, **dadurch gekennzeichnet, dass** Dichlormethan als Extraktionsmittel verwendet wird.

10. Verfahren nach Anspruch 8, **dadurch gekennzeichnet, dass** das Kristallisationslösungsmittel Acetonitril oder Toluol, oder deren Mischungen, ist.

## Revendications

1. Un procédé de préparation de bromure de tiotropium de formule **II**:

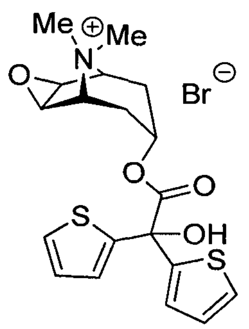

**II**

**caractérisé en ce qu'**il comprend les étapes suivantes:

a) préparation de l'ester de scopine de formule **I**

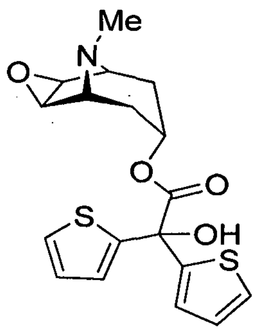

**I**

par transestérification de méthyl-di(2-thiényl)-glycolate de formule IV avec une scopine de formule III

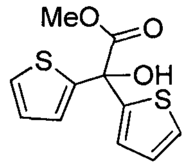

IV

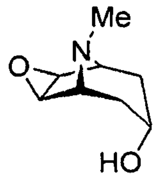

III

en présence d'une quantité sous-stoechiométrique d'une base stériquement encombrée choisie dans le groupe des sels alcalins d'alkoxydes ramifiés en $C_3$ à $C_5$ dans un solvant inerte,
b) isolement de l'ester de scopine de formule I, et
c) quaternisation de l'ester de scopine de formule I avec du bromure de méthyle.

2. Le procédé selon la revendication 1, **caractérisé en ce que** la base stériquement encombrée est choisie dans le groupe des sels de sodium ou de potassium de *tert*-butoxyde, *tert*-pentoxyde et isopropoxyde.

3. Le procédé selon la revendication 2, **caractérisé en ce que** la base stériquement encombrée est le sel de sodium ou de potassium de *tert*-butoxyde ou de *tert*-pentoxyde.

4. Le procédé selon la revendication 1, **caractérisé en ce que** la base stériquement encombrée est utilisée dans une proportion de 0,3 à 0,7 équivalents par rapport à la scopine.

5. Le procédé selon la revendication 1, **caractérisé en ce que** le toluène, le tétrahydrofurane ou leurs mélanges sont utilisés en tant que solvants inertes.

6. Le procédé selon les revendications 1-5, **caractérisé en ce que** la transestérification est effectuée à une température dans la plage de 80 à 130°C.

7. Le procédé selon la revendication 6, **caractérisé en ce que** la transestérification est effectuée à une pression dans la plage de 50 kPa à 30 kPa, le méthanol formé étant simultanément éliminé par distillation.

8. Le procédé selon la revendication 1, **caractérisé en ce que** l'isolement de l'étape (b) comprend

- le mélange du mélange réactionnel issu de l'étape (a), éventuellement après dilution avec un solvant d'extraction, dans une solution aqueuse d'un acide inorganique,
- l'extraction de la phase aqueuse avec un solvant d'extraction,
- l'alcalinisation de la phase aqueuse avec une base inorganique à un pH dans la gamme de 7-9,
- l'extraction du produit avec un solvant d'extraction et,
- après concentration, la cristallisation de l'ester de scopine brut de formule I à partir du solvant de cristallisation.

9. Le procédé selon la revendication 8, **caractérisé en ce que** le dichlorométhane est utilisé comme agent d'extraction.

10. Le procédé selon la revendication 8, **caractérisé en ce que** le solvant de cristallisation est l'acétonitrile ou le toluène ou leurs mélanges.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0418716 A
- US 6506900 B1
- US 6747154 B2
- US 20060047120 A
- WO 2008008376 A
- WO 2009087419 A
- EP 041876 A
- EP 0418714 A